# EUROPEAN PATENT APPLICATION

(11) **EP 2 556 807 A2**
(43) Date of publication of application: **13.02.2013**
(21) Application number: 11766153.8
(22) Date of filing: 06.04.2011
(51) Int. Cl.: A61F 2/24, A61L 27/14, A61M 1/10

(54) **POLYMER VALVE AND PULSATILE CATHETER-TYPE VENTRICULAR ASSIST DEVICE USING SAME**

(30) Priority: 06.04.2010 KR 20100031234
(71) Applicant: Libraheart Inc., Jeju-do 690-756 (KR)
(72) Inventor: MIN, Byoung Goo, Goyang-si Gyeonggi-do 410-382 (KR); LEE, Jung Chan, Seoul 136-090 (KR); WON, Yong Soon, Seoul 132-030 (KR); KIM, Wha Ryong, Seoul 136-037 (KR); SHIN, Pil Kyeong, Jeju-si Jeju-do 690-811 (KR)
(74) Representative: Beier, Ralph
(86) International application number: PCT/KR2011/002414
(87) International publication number: WO 2011/126302

(57) **Abstract**

Disclosed are a polymer valve and a ventricular assist device using the polymer valve. The polymer valve, according to the present invention, allows blood to flow in a first direction but prevents blood from flowing backward in a second direction opposite to the first direction. The polymer valve comprises a base body having a hollow therethrough for passing of blood, comprising a pair of support legs formed by cutting opposing sides of an end portion of the base body along an inclined surface toward the center line of the hollow to extend toward the first direction from the body , and a leaflet formed of polymer and attached to the support legs to cover the hollow, wherein the leaflet includes a pair of opposing sides attached to the cutting surfaces of the support legs and an end portion connecting the sides of the leaflet, wherein the leaflet is formed of an opening between the support legs, whereby the leaflet allows blood to pass through the opening toward the first direction and prevent blood from flowing through the opening toward the second direction by the contact of the opposing sides of the leaflet around the opening.

## Description

### TECHNICAL FIELD

This invention relates to a polymer valve a ventricular assist device (VAD) using the polymer valve, and more particularly to an improved polymer valve and a pulsatile conduit-type ventricular assist device using the polymer valve to substantially expand a device life span and durability.

### BACKGROUND

Heart valves maintain the unidirectional flow of blood in the heart by opening and closing depending on the difference in pressure on each side. A human heart carries four valves: a bicuspid valve between a right atrum and a right ventricle; a pulmanary valve between a pulmonary artary and a right ventricle; an aortic valve between an aorta and a left ventricle; and a mitral valve between a left atrium and a left ventricle. In recent years, artificial heart valves have been developed to replace malfunctionling heart valves. Artificial heart valves include mechanical, biological and polymer valves.

Mechanical valves are devices opening unidirectional and formed of metal alloy that does not harm to blood cells. One of the major drawbacks of mechanical heart valves is that patients with these implants require consistent anti-coagulation therapy. Clots formed by red blood cell and platelet damage can block up blood vessels and lead to very serious consequences. Also, noises that occur during opening and closing of the mechanical valves were suffering to patients.

In order to overcome the limitation of such mechanical heart valves, biological valves are introduced in 1969. Biological valves are valves of animals, like pigs, which undergo several chemical procedures in order to make them suitable for implantation in the human heart. There are some risks associated with a biological valve such as the human body's tendency to reject foreign material, thus resulting in a replacement implantation in about ten to fifteen years.

A recent introducion is a polymer valve which is cost effective compared to mechanical or biological valve and enables fabrication of a desired shape with ease. A polymer valve varies to a bi-leaflet and tri-leaflet type.

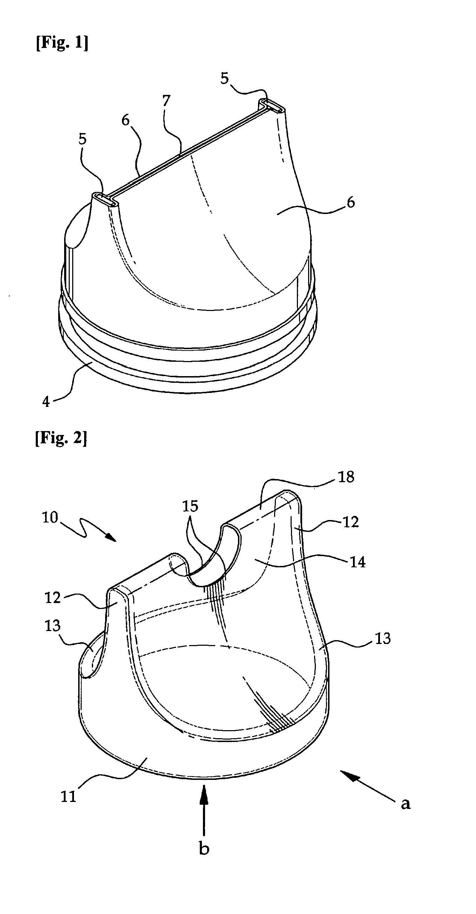

Fig. 1 illustrate conventional bi-leaflet polymer valve. With reference to Fig. 1, the polymer valve includes a support body 4 having a pair of support legs 5 and a leaflet 6 formed of polymer surrounding the support body 4. The end portion of the leaflet 6 separated by a slit 7 of a line shape. When blood flow in a first direction, the slit is opened. When blood flows in a second direction, the slit closed by a pressure applied to the outer surface of the leaflet.

Meanwhile, Ventricular Assist Device (VAD) is used for a patient having heart failure when it is difficult to cure the disease by open heart surgery or there is no effect with medical treatments. Also, VAD is used temporally in order to substitute a ventricle before heart transplantation or in order to reduce a load to heart for the sake of recovery.

Ventricular Assist Devices can be classified into an implantable type VAD and an extracorporeal type VAD according to the mounted place. Also, Ventricular Assist Devices can be divided into a left ventricle VAD (LVAD) and a right ventricle VAD (RVAD) and dual ventricles VAD (BiVAD) according to the ventricles assisted. Most of the VADs used are LVAD. Also, Ventricular Assist Devices can be classified into pneumatic type VAD and electric type VAD according to the kinds of power supply. Electric type VAD can be classified into an electrohydraulic type VAD and an electromechanical type VAD. Also, Ventricular Assist Devices can be classified into a pulsatile type VAD and a nonpulsatile type VAD according to the generation of pulsation for driving blood to flow. A implantable type BiVAD may be regarded as an artificial heart, but it is different from a complete artificial heart that replaces a natural heart and performs circulation of blood.

Among various types of VADs, most of them are VADs using axial type blood pump that includes rotary pump or impeller. But these kinds of VADs have drawbacks such that thrombus occurs during use because blood contact with the surface of metal part of a component and blood cannot flow through the device when the device does not work. Also, these kinds of VADs are very expensive.

To overcome the problems of the VADs using axial type blood pump, pulsatile pumps that have two artificial valves and operated by use of pneumatic power was developed. U.S. patent No. 6,579,223 (title of the invention "BLOOD PUMP") discloses that kind of pump.

Meanwhile, in polymer valves described above blood flows through a narrow slit formed between leaflets so it is difficult for the slit to flow enough quantity of blood. Also, leaflets repeatedly moves back and forth toward each other such that repeatedly push and pull the support legs to be bent back and forth. Therefor the support legs tend to break by fatigue and the leaflet are apt to be taken off from the support base. These may cause the reduction of durability of VADs.

In VADs using polymer valves, it is necessary to use polymer valves that have long durability and especially can bear a strong backflow pressure. As explained above, conventional polymer valves may cause break down of the VADs by a repeated strong backflow pressure such as separation of leaflets from the support legs and rupture of the leaflets. Therefor it is needed to replace VAD or valves. The replacement of VAD or valves means an additional cost to a patient and lack of durability.

The present invention is contrived to overcome the conventional disadvantages. Accordingly, an objective of the present invention is to provide a polymer valve to evenly distribute force and pressure applied to the leaflets to improve a valve life span and durability.
Another objective is to provide a polymer valve for a VAD with a cost-effective simplified structure to save more patients suffering from blood valve malfunctioning.

### SUMMARY OF THE INVENTION

According to the present invention, a polymer valve for allowing blood to flow toward a first direction and preventing blood from flowing toward a second direction opposite to the first direction is provided. The polymer valve comprising; a base body having a hollow therethrough for passing of blood, comprising a pair of support legs formed by cutting opposing sides of an end portion of the base body along an inclined surface toward the center line of the hollow to extend toward the first direction from the body; and a leaflet formed of polymer and attached to the support legs to cover the hollow, wherein the leaflet includes a pair of opposing sides attached to the cutting surfaces of the support legs and an end portion connecting the sides, wherein the leaflet is formed of an opening between the support legs, whereby the leaflet allows blood to pass through the opening toward the first direction and prevent blood from flowing through the opening toward the second direction by the contact of the opposing sides of the leaflet around the opening.

In some embodiments, the opening of the polymer valve may extend from a side of the leaflet to the opposing side of the leaflet through the end portion of the leaflet.

Also, the opening may be substantially shaped in a letter 'U' when viewed from a cutting surface.

According to the present invention, a ventricular assist device for pumping blood is provided. The ventricular assist device, comprising; a conduit, a pair of polymer valves installed within the conduit to allow blood to flow toward a first direction and prevent blood from flowing toward a second direction opposite to the first direction, and a housing to enclose the conduit to form a hermetic space outside of the conduit; wherein each of the polymer valves comprises, a base body having a hollow therethrough for passing of blood, comprising a pair of support legs formed by cutting opposing sides of an end portion of the base body along an inclined surface toward the center line of the hollow to extend toward the first direction from the body , and a leaflet formed of polymer and attached to the support legs to cover the hollow, wherein the leaflet include a pair of opposing sides attached to the cutting surfaces of the support legs and an end portion connecting the sides, wherein the leaflet is formed of an opening between the support legs, whereby the leaflet allows blood to pass through the opening toward the first direction and prevent blood from flowing through the opening toward the second direction by the contact of the opposing sides of the leaflet around the opening. The ventricular assist device configured to pump blood toward the first direction by a change of an air pressure inside the housing.

In some embodiments, the conduit of the device may be formed of an elastic material.

In some embodiments, each opening of the polymer valves of the device may extend from a side of the leaflet to the opposing side of the leaflet through the end portion of the leaflet.

In some embodiments, each opening of the polymer valves of the device may be substantially shaped in a letter `U' when viewed from a cutting surface.

The polymer valve of the present invention can have longer durability because the opening serves to evenly distribute a blood flow pressure and force to the entire surface of the singular leaflet. Also, the polymer valve can provide larger blood flow rate compared to the conventional polymer valves according to the shape of the opening.

Also, the ventricular assist device using the polymer valve can have longer durability. Moreover the ventricular assist device of the present invention can be used as a bypass conduit when an air pump does not work so the device can be helpful to the treatment of heart failure. Also, the ventricular assist device of the present invention can provide various kinds of treatments for the heart failure patients such as treatment using an air pump or treatment not using an air pump.

### BRIEF DESCRIPTION OF THE DRAWINGS

FIG. 1 is a perspective view of a conventional bi-leaf polymer valve;

FIG. 2 is a perspective view showing a polymer valve according to the present invention;

FIG. 3 is a perspective view showing a base body of a polymer valve according to the present invention;

FIG. 4 is a perspective view showing preferred embodiments of a polymer valve having different opening shapes according to the present invention;

FIG. 5 shows molding steps to fabricate a polymer valve according to the present invention;

FIG. 6 is a schematic prospective view showing a ventricular assist device using polymer valves according to the present invention; and

FIG. 7 shows mechanism of blood flow through the ventricular assist device according the present invention.

### DETAILED DESCRIPTION OF THE PREFERRED EMBODIMENTS

Referring to the accompanying drawings, preferred embodiments of the present invention will now be explained.

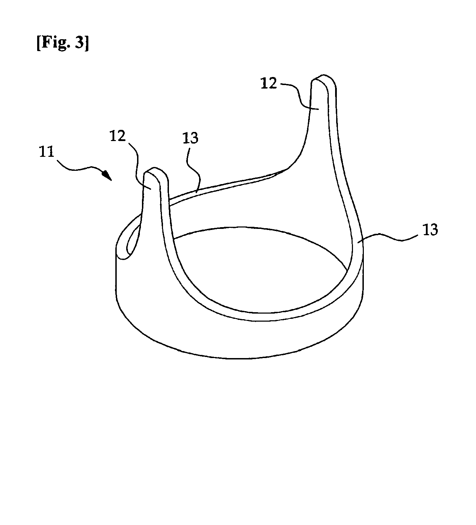

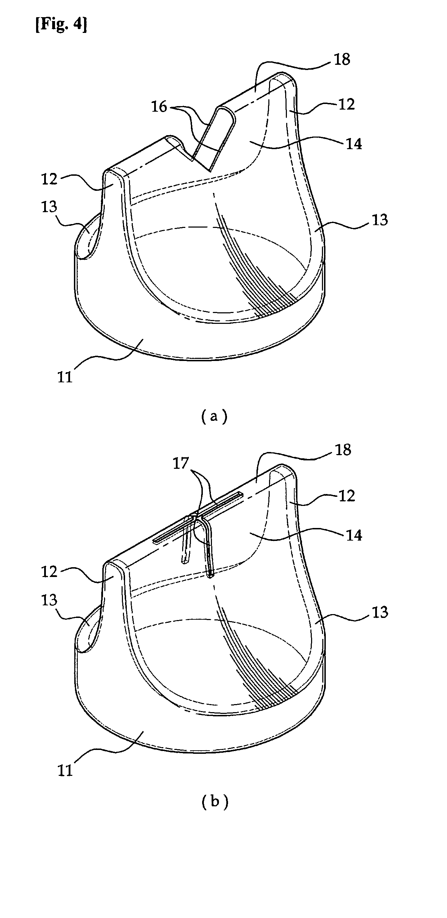

FIG. 2 is a perspective view showing a polymer valve according to the present invention, and FIG. 3 is a perspective view showing a base body of a polymer valve according to the present invention, and FIG. 4 is a perspective view showing preferred embodiments of a polymer valve having different opening shapes according to the present invention. Referring to FIG. 2 and 3, a polymer valve 10 comprises a base body 11 and a leaflet 14.

As shown in FIG. 3, the base body 11 having a hollow therethrough for the flow of blood and includes a pair of support legs 12. Each support leg 12 is extended from the opposing sides of the base body 11 in symmetry. Each support leg 12 can be formed by cutting opposing sides of an end portion of the base body 11 along an inclined surface toward the center line of the hollow. The inclined surface may be formed by cutting opposing sides of a hollow cylinder pipe, so the inclined surface may be cutting surface 13. If the support legs 12 are in symmetry, the cutting surface 13 may be in symmetry. The support legs 12 support the leaflet 14. The support legs 12 may be formed by a material proved for medical use such as polycarbonate or polyurethane for medical use.

In an embodiment, the leaflet 14 is formed of a polymer film. The leaflet 14 may be formed of a polyurethane film. The leaflet 14 attached to and along the cutting surface 13 and the tops of the support legs 12 to cover the hollow. The leaflet includes a pair of opposing sides attached to the cutting surfaces 13 of the support legs 12 and an end portion 18 connecting the sides of the leaflet. An opening 15 is formed on the leaflet and between the support legs 12. The opening 15 of the leaflet 14 extends from a side of the leaflet to the opposing side of the leaflet through the end portion of the singular leaflet. A side and the opposing side of the leaflet can moves back and forth, which results in a refined mechanism in which a forward or outward blood flow passes through the opening 40 (arrow b direction in FIG. 2) and a reverse flow or backflow of blood presses opposing leaflet sides around the opening 15 to contact each other and thereby prevents the blood backflow.

The opening 15 is substantially shaped in a letter 'U' when viewed from a side of the cutting surface 13 (arrow a direction in FIG. 2). U shape of the opening enables the polymer valve to flow higher blood flow rate. Also, the polymer valve 10 can evenly distribute a blood flow pressure and force to the leaflet 14. Distribution of force to the leaflet brings long durability to the polymer valve.

Selectively, the opening may be formed in a V-shape 16 as shown in FIG. 4A, or in a slit 17 (T letter shape) as shown in FIG. 3B. The format of the leaflet opening may vary depending on a target patient and medical record of the patient so as to effectively control the amount of the unit blood flow.

With reference to FIG. 2, the working mechanism of the polymer valve 10 will now be further explained.

When blood flows toward arrow b direction in FIG. 2, blood pushes opposing sides of the leaflet 14 from inside of the hollow to the outside of the hollow. Therefore the opposing sides of the leaflet separated apart, the opening 15 of the leaflet opens, and blood can flow through the opening 15. When blood flows toward arrow b direction, the opposing sides of the leaflet 14 are in open position.

When blood tends to flow toward reverse direction of the arrow b, blood pushes opposing sides of the leaflet 14 from outside of the hollow to the inside of the hollow. The opposing sides of the leaflet 14 around the opening 15 contact each other, therefor the opening 15 closed. When blood tends to flow toward reverse direction of the arrow b, the opposing sides of the leaflet 14 are in closed position.

The leaflet can be formed of a flexible material to be movable between the open position and the closed position. In the polymer valve of the present invention, the force applied to the leaflet by the blood pressure can be distributed evenly to the leaflet 14 around the opening. Therefor the support legs do not get concentrated load by the leaflet 14, which results in long durability of the polymer valve by preventing the separation of the leaflet 14 from the support legs and fatigue failure.

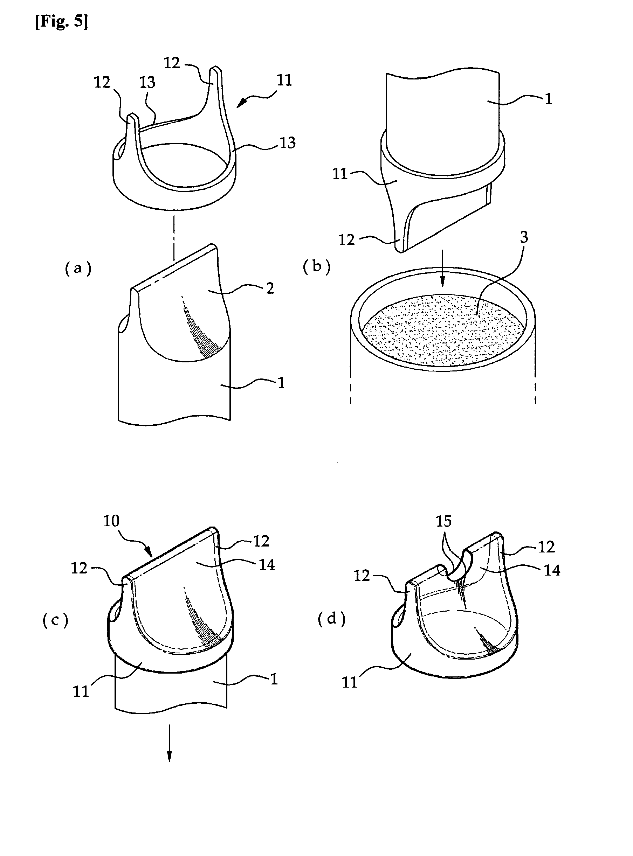

FIG 5 shows molding steps to form the polymer valve 10, which will be further explained below.

Fabrication steps of the blood valve 10 are illustrated in FIGS. 5(a) to 5(d). As shown FIG 5(a), a mold 1 is formed and inserted in the base body 11 through the hollow. The mold 1 has opposing curved sides 2 corresponding to the leaflet 14 to align with the cutting surfaces 13 of the body 11.

Then, as shown in FIG. 5(b), the mold 1 with the body 11 is dipped in a polymer liquid 3 and taken out for dry using a dip casting method to laminate the mold 1 and the outer surface of the base body 11 with the polymer. This dip casting step may be repeated with a predetermined interval to improve quality and durability of the leaflet 14.

As shown in FIG. 5(c), the mold 1 is detached from the base body 11 and the opening 15 is formed by partially cutting out a central portion of the leaflet using a known cutting tool to fabricate the blood valve 10 with the leaflet 14 as shown FIG. 5(d).

When the polymer valve opens and closes, no concentrated force applied to the leaflet of the polymer valve of the present invention. All the forces applied to the leaflet distributed evenly to the surfaces of the leaflet, therefore the polymer valve can have long durability. The merits of the polymer valve can be applied to the ventricular assist device using the polymer valves of the present invention.

Hereinafter, a ventricular assist device using the polymer valve 10 will be explained.

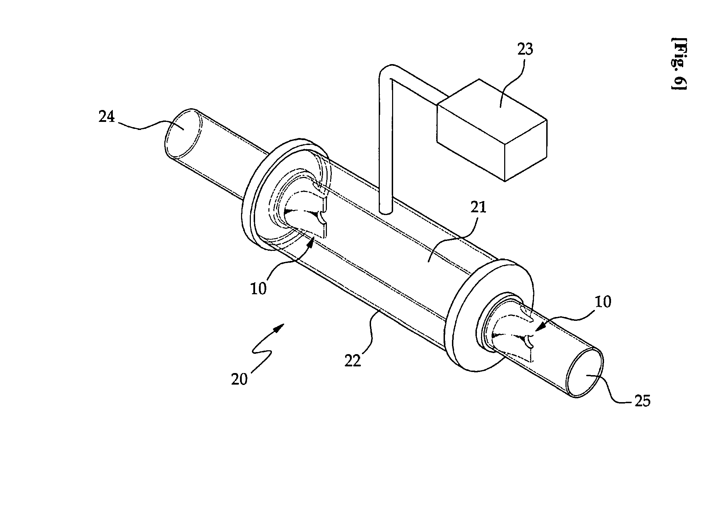

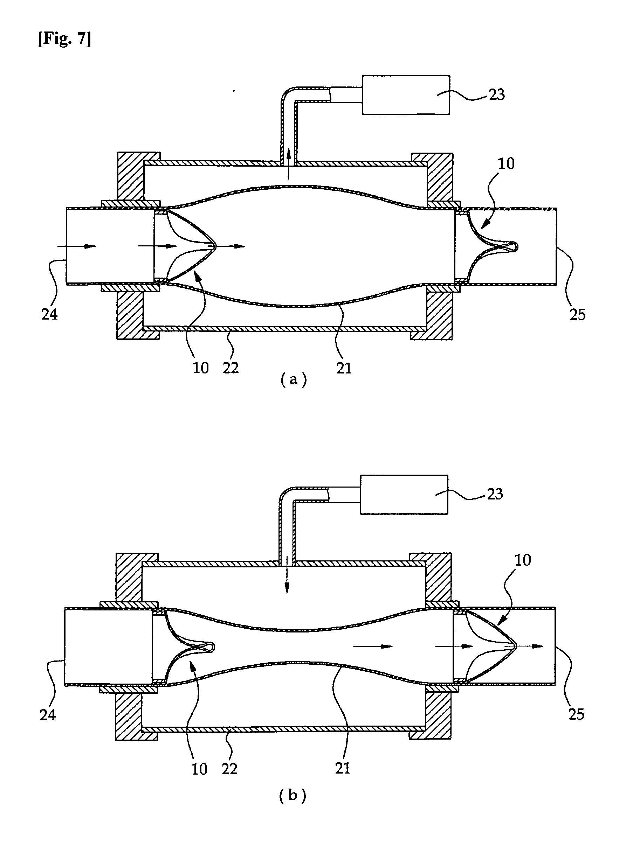

FIG. 6 is a schematic prospective view showing a ventricular assist device using polymer valves according to the present invention, and FIG. 7 shows mechanism of blood flow through the ventricular assist device according the present invention. With reference to FIG. 6 and FIG. 7, a ventricular assist device 20 comprises a conduit 21, a pair of polymer valves 10, a housing 22, and an air pump.

The conduit 21 is hollow and long that blood can flow through the conduit. The conduit 21 can be a flexible material. The elastic conduit able to contract and expend is desirable so that the conduit 21 can be served as an apical aortic conduit (AAC) when the ventricle assist device is not used as a ventricle assist device. Also, it is desirable to form the conduit 21 with a soft bio-friendly polymer material, preferably, a medical-purpose polyurethane. A pair of polymer valves 10 is installed to the inlet and outlet of the conduit 21 only to allow blood to flow toward a direction.

The housing 22 is installed to enclose the conduit to form a hermetic space outside of the conduit. It is desirable to form the housing 22 with a hard bio-friendly polymer material. Also it is more desirable to form the housing with a transparent material to be seen inside of the housing.

The air pump 23 is coupled with the housing to provide an air into the hermetic space between the housing 22 and the conduit 21. Since there are many kinds of air pump available commercially, it does not need to explain the air pump in detail.

Hereinafter, with reference to FIG. 6 and 7, the working mechanism of the ventricular assist device will be explained in further detail.

The entrance 24 serves to communicate with a ventricle of a patient and the exit 25 communicates with an aorta of the patient. When the air inside the housing 22 is suctioned out by using the air pump 23, the conduit 21 comes to inflate or expand since the lowered pressure in the housing 22 triggers inflation of the conduit 21 as shown in FIG. 7(a). Consequently, the inflation of the conduit 21 causes the blood to flow in through the polymer valve 10 installed in entrance 24 side with the polymer valve 10 installed at exit 25 side 80 serving to block backflow.

As shown in FIG. 7B, when the air pump 23 provide an air into the housing 22 then the increased air pressure inside the housing 22 leads to deflate the conduit 21 as much thereby moving the blood inside the conduit 21 through the polymer valve installed in exit 25 side to the exit 25 with the polymer valve 10 installed in entrance 24 side serving to block backflow.

As explained above, blood flows in and out the ventricular assist device by cyclic processes of suctioning and injecting an air into the housing by the air pump. By the blood pumping process of the device like that, the device reduces loads of ventricle and can be used to the treatment of heart failure. Especially, since the polymer valve has long durability, also the ventricular assist device will have long durability.

Also, in an embodiment, the conduit 21 can be served as an apical aortic conduit (AAC). When a ventricle pumps out blood, then the pumped out blood flows into the conduit 21 and the blood flows out through the polymer valve 10 installed at the exit 25 side to the exit 25 which communicates with an aorta. The polymer valve 10 installed at the entrance 24 side blocks backflow of the blood. This will decrease load to the ventricle thereby curing a heart failure. A highly elastic material for the conduit 21 would maximize an effective decrease of load to the ventricle.

As explained above, when using an air pump, the ventricular assist device can actively reduce load to a ventricle. On the other hand, without the air pump, the ventricular assist device can be used as an apical aortic conduit to bypass the blood from ventricle to aorta. Malfunction of a blood pump using an impeller or rotary pump can be fatal to a patient because the blood cannot pass the blood pump. But the ventricular assist device 20 according to the present invention can be served as AAC, therefore in the event of the malfunctioning of the air pump 23 the device 20 enables blood to pass through it, thereby the device 20 would not affect the heart functioning of the patient fatally. Further, when the ventricular assist device 20 is provided such that it could be implanted, the patient can manually connect the air pump 23 to the housing 23 at home and detach the air pump 23 for the patient to go out for an outdoor activity, thereby minimizing the inconvenience of living and maximizing the effect of treatment of heart failure.

Although the invention has been described in considerable detail with reference to certain preferred embodiments, other embodiments are possible by converting the aforementioned construction. Therefore, the scope of the invention shall not be limited by the embodiments specified above.

## Claims

1. A polymer valve for allowing blood to flow toward a first direction and preventing blood from flowing toward a second direction opposite to the first direction, the polymer valve comprising;
a base body having a hollow therethrough for passing of blood, comprising a pair of support legs formed by cutting opposing sides of an end portion of the base body along an inclined surface toward the center line of the hollow to extend toward the first direction from the body , and
a leaflet formed of polymer and attached to the support legs to cover the hollow, wherein the leaflet includes a pair of opposing sides attached to the cutting surfaces of the support legs and an end portion connecting the sides of the leaflet, wherein the leaflet is formed of an opening between the support legs, whereby the leaflet allows blood to pass through the opening toward the first direction and prevent blood from flowing through the opening toward the second direction by the contact of the opposing sides of the leaflet around the opening.

2. The polymer valve of claim 1, wherein the opening extends from a side of the leaflet to the opposing side of the leaflet through the end portion of the singular leaflet.

3. The polymer valve of claim 2, wherein the opening is substantially shaped in a letter 'U' when viewed from a cutting surface.

4. A ventricular assist device, comprising;
a conduit,
a pair of polymer valves installed within the conduit to allow blood to flow toward a first direction and prevent blood from flowing toward a second direction opposite to the first direction,
a housing to enclose the conduit to form a hermetic space outside of the conduit,
wherein each of the polymer valves comprises,
a base body having a hollow therethrough for passing of blood, comprising a pair of support legs formed by cutting opposing sides of an end portion of the base body along an inclined surface toward the center line of the hollow to extend toward the first direction from the body , and
a leaflet formed of polymer and attached to the support legs to cover the hollow, wherein the leaflet includes a pair of opposing sides attached to the cutting surfaces of the support legs and an end portion connecting the sides, wherein the leaflet is formed of an opening between the support legs, whereby the leaflet allows blood to pass through the opening toward the first direction and prevent blood from flowing through the opening toward the second direction by the contact of the opposing sides of the leaflet around the opening,
whereby the ventricular assist device configured to pump blood toward the first direction by a change of an air pressure inside the housing.

5. The ventricular assist device of claim 4, wherein the conduit is formed of an elastic material.

6. The ventricular assist device of claim 4, wherein each opening of the polymer valves extends from a side of the leaflet to the opposing side of the leaflet through the end portion of the singular leaflet.

7. The ventricular assist device of claim 6, wherein each opening of the polymer valves is substantially shaped in a letter 'U' when viewed from a cutting surface.
